Europäisches Patentamt

European Patent Office

Office européen des brevets

(11) Veröffentlichungsnummer: **0 402 681**

**A1**

## (12) EUROPÄISCHE PATENTANMELDUNG

(21) Anmeldenummer: **90109966.3**

(22) Anmeldetag: **25.05.90**

(51) Int. Cl.5: **H01R 13/24, H01R 17/18**

(30) Priorität: **08.06.89 CH 2145/89**

(43) Veröffentlichungstag der Anmeldung:
**19.12.90 Patentblatt 90/51**

(84) Benannte Vertragsstaaten:
**AT BE DE DK ES FR GB IT NL SE**

(71) Anmelder: **ASCOM AUDIOSYS AG**
**Bernstrasse 41**
**CH-3175 Flamatt(CH)**

(72) Erfinder: **Renggli, Peter**
**Schlossstrasse 20**
**CH-3098 Köniz(CH)**

(74) Vertreter: **Scheidegger, Werner & Co.**
**Stampfenbachstrasse 48 Postfach**
**CH-8023 Zürich(CH)**

(54) **Elektrischer Steckverbinder.**

(57) Die elektrische Steckverbindung besitzt im Steckernegativ Kontaktformstücke (8,9) aus elektrisch leitendem Elastomer. Diese umfassen die Leiterflächen (2a,2b) des Steckerpositivs (1) und gewährleisten damit eine zuverlässige elektrische Verbindung bei kleinsten Abmessungen.

Fig.1

EP 0 402 681 A1

## Elektrischer Steckverbinder

Die vorliegende Erfindung betrifft ein Steckernegativ für elektrischen Steckverbinder, insbesondere für miniaturisierte Bauteile, zur Aufnahme eines als Stift ausgebildeten mindestens einpoligen Steckerpositivs, an dessen Aussenfläche wenigstens eine Leiterfläche vorgesehen ist, wobei das Steckernegativ für den jeweiligen Kontakt mit der Leiterfläche bzw. Leiterflächen eines Stekkerpositivs ausgebildet ist, sowie ein Steckerpositiv für elektrischen Steckverbinder mit einem wenigstens eine Leiterfläche aufweisenden Kontaktstift, zur Verwendung mit einem Steckernegativ der vorgenannten Art.

Derartig aufgebaute Steckverbinder sind in verschiedenen Ausführungsformen und Grössen bekannt und werden insbesondere in miniaturisierter Form im Audio-Bereich in grosser Anzahl verwendet. Insbesondere im Miniatur-Bereich treten allerdings Probleme auf, welche einer extremen Miniaturisierung entgegenstehen. Diese sind beispielsweise in der Lage und Masstoleranz der Steckerpartner, im Aufbringen des für die elektrisch leitende Verbindung notwendigen Kontaktdruckes sowie bei der Bildung von isolierenden Fremdschichten, beispielsweise durch Verschmutzung oder Bakterien, zu finden.

Das Erzeugen eines sicheren Kontaktdruckes auf die meist punktförmige metallische Verbindungszone zwischen Steckerpositiv und Steckergativ erfordern bei der Miniaturisierung einen ausserordentlichen technischen Aufwand, ebenso die Beherrschung der Form- und Lagetoleranzen. Dies führt insbesondere im Verhältnis zur mögli chen Verkleinerung des Steckerdurchmessers zu grossen Steckernegativkonstruktionen und damit wieder zu einem entsprechend grossen Platzbedarf im damit auszurüstenden Gerät.

Aufgabe der vorliegenden Erfindung ist es nun, diesen Problemen abzuhelfen und damit insbesondere eine weitere Verkleinerung von Steckverbindern zu ermöglichen.

Diese Aufgabe wird bei einem Steckernegativ der eingangs definierten Art erfindungsgemäss dadurch gelöst, dass das Steckernegativ ein Isolatorgehäuse aufweist, welches wenigstens ein leitendes Anschluss- bzw. Kontaktelement trägt und das Ganze mit einer ggf. durchgehenden Ausnehmung zur Aufnahme des Stiftes eines Steckerpositivs versehen ist, wobei jedes Anschluss- bzw. Kontaktelement mit wenigstens einem in die Ausnehmung ragenden Kontaktformstück aus elektrisch leitendem Elastomer verbunden ist.

Vorzugsweise sind zwecks Aufnahme eines Steckerpositivs mit mehreren elektrisch voneinander getrennten Leiterflächen mehrere, z.B. zwei elektrisch voneinander getrennte leitende Anschluss- bzw. Kontaktelemente vorgesehen.

Es ist besonders zweckmässig, dass jedes Kontaktformstück als scheiben- oder ringförmiges Element mit einem Durchgang bzw. einer Ausnehmung zur Aufnahme des Stiftes eines Steckerpositivs ausgebildet ist. Bei derartigen Ausführungsformen ist es vorteilhaft, dass die Kontaktformstücke hintereinander angeordnet sind und dass deren Durchgänge bzw. Ausnehmungen zueinander ausgerichtet sind.

Es ist angezeigt, dass der freie Querschnitt der Ausnehmungen in den Kontaktformstücken kleiner ist als der kontaktwirksame Querschnitt des aufzunehmenden Stiftes des Steckerpositivs.

Es ist auch möglich, dass der Querschnitt der Ausnehmungen in den Kontaktformstücken unrund, z.B. vieleckig ausgebildet ist.

Bei einer besonders zweckmässigen Ausführungsform bestehen die Kontaktformstücke aus elektrisch leitendem Silicon-Elastomer, z.B. aus einem mit leitendem amorphem Köhlenstoff dotierten, hochtemperaturvulkanisierten Silicon-Elastomer.

Ebenfalls Gegenstand der Erfindung bildet ein Steckerpositiv für elektrischen Steckverbinder mit einem wenigstens eine Leiterfläche aufweisenden Kontaktstift, zur Verwendung mit einem Steckernegativ, welches sich dadurch auszeichnet, dass der Kontaktstift einen unrunden, z.B. einen vieleckigen Querschnitt aufweist. Dieses Steckerpositiv lässt sich durch Verdrehen um einen bestimmten Winkel, z.B. 90°, in guten Kontakt mit den leitenden Kontaktformstücken des Steckernegativs bringen.

Dadurch dass sich das Kontaktformstück beim Einführen des Stiftes deformieren kann, ist die Anforderung an die exakte Lage dieses Steckerteiles wesentlich verkleinert worden. Die elastische Aufnahme des Stiftes lässt gewisse kleine Toleranzen bezüglich Lage und Winkel in radialer Richtung zu. Die Positionierung in axialer Richtung erfolgt wie üblich durch einen Anschlag, gegen welchen der Stift gestossen wird.

Nach dem Einfügen des Stiftes in das Steckernegativ werden dessen Kontaktzonen vom Kontaktformstück grossflächig umschlossen. Insbesondere bei kleinerem Durchmesser der Ausnehmung der Kontaktformstücke gegenüber dem Steckerdurchmesser entsteht ein inniger Reibschluss. Durch die Verwendung von Elastomer als Material für die Kontaktformstücke entsteht ein allseitiger Druck auf den umschlossenen Steckerstift. Elektrische Signale können nun von der Kontaktfläche des Steckerstiftes über das Kontaktformstück an einen an dieses anzuschliessenden Leiter übertragen werden.

Durch axiale Anordnung von mehreren, elektrisch voneinander isolierten Kontaktformstücken im Isolatorgehäuse des Steckernegativs kann bei entsprechender Ausbildung des Steckerstiftes eine praktisch beliebige Anzahl von elektrischen Verbindungen mit einer Steckerverbindung erzielt werden.

Die radialen Abmessungen dieser Steckerverbindung können sehr klein gewählt werden, da nicht nur eine punktförmige, sondern eine auf den gesamten Umfang des vom Kontaktformstück umschlossenen Bereiches des Steckerstiftes wirksame Kontaktstelle gebildet wird.

Ebenfalls nimmt die Einbaugrösse des gesamten Steckernegativs ab, da keine viel Raum beanspruchende metallische Federelemente verwendet werden müssen. Da die Anforderungen an die Positionierung durch die erfindungsgemässe Ausgestaltung kleiner geworden sind, lässt sich das Steckernegativ freier wählbar im Innern eines Gerätes platzieren. Damit kann das Steckernegativ vorteilhaft auch als oberflächenmontierbares Bauteil (SMD) ausgestaltet werden und erleichtert damit die Herstellung von Geräten,welche eine derartige Steckerverbindung benötigen.

Durch die Reibung beim Einführen und Entnehmen des Steckerstiftes wird zusätzlich ein Selbstreinigungseffekt der Leiterflächen sowohl am Stift des Steckerpositivs als auch an den Kontaktformstücken des Steckernegativs erreicht.

Besonders vorteilhaff erweist sich auch die unrunde Gestaltung des Steckerstiftquerschnittes und des Ausnehmungsquerschnittes der Kontaktformstücke. Damit wird der Kontaktdruck durch axiales Verdrehen nach dem Einführen des Steckerstiftes erhöht.

Ausführungsbeispiele der Erfindung werden nachstehend anhand der Zeichnung noch etwas näher erläutert. Es zeigen:

Fig. 1 einen Axialschnitt durch eine zweipolige Steckerverbindung, mit einem erfindungsgemässen Steckernegativ und einem als Stift ausgebildeten Steckerpositiv, und

Fig. 2 einen Querschnitt durch ein Kontaktformstück, in welches ein unrundes Steckerpositiv einzuführen ist (Funktionsprinzip, rein schematisch).

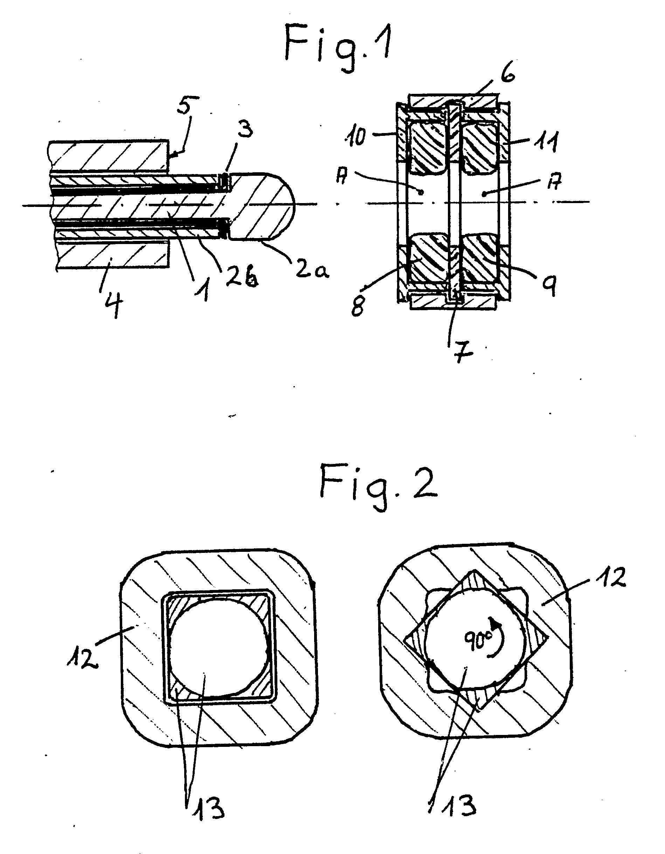

Fig. 1 zeigt einen Teil eines Steckerpositivs 1, bestehend aus einem ersten metallischen Anschlussstück 2a, einem zweiten Anschlussstück mit Anschlussfläche 2b, einer elektrisch trennenden Isolierschicht 3 sowie einer Steckerumhüllung 4 mit die Einschiebtiefe begrenzender Anschlagfläche 5.

Das zugehörige Steckernegativ weist ein umhüllendes Isolatorgehäuse 6, zwei durch eine Isolierscheibe 7 elektrisch voneinander getrennte Kontaktformstücke 8,9 auf, welche in metallischen Anschlussstücken 10,11 eingefasst sind. Das eine Anschlussstück 10 dient hier zugleich als axialer Gegenanschlag für den Stecker 1, wenn der Stecker 1 bis zum Kontakt der Anschlagfläche 5 mit dem Anschlussstück 10 in das Steckernegativ eingeschoben wird.

Nach dem Einführen des Steckers 1 sind das Anschlussstück 2a mit dem Kontaktformstück 9 bzw. die Anschlussfläche 2b mit dem Kontaktformstück 8 elektrisch leitend verbunden. Die elektrisch leitende Verbindung mit dem Gerät erfolgt über die metallischen Anschlussstücke 10,11.

Die eigentlichen Kontaktformstücke 8,9 bestehen aus elektrisch leitendem Silicon-Elastomer, insbesondere aus mit amorphem Kohlenstoff dotiertem, hochtemperaturvernetztem Silicon-Elastomer. Dieses Material weist einen spezifischen Durchgangswiderstand von 2-20 Ohm·cm, eine Belastbarkeit von 30 V/3mA und eine Härte von ca. 50 Shore A auf. Zudem ist das Material löttemperaturfest (300°, 20 Sek.).

Fig. 2 zeigt rein schematisch ein Kontaktformstück 12 für ein Steckernegativ mit quadratischem Durchgang, sowie den in einem bestimmten Bereich im Querschnitt ebenfalls quadratischen Stift 13 eines Steckerpositivs.

Durch Verdrehen um 90° des Stiftes 13 mit unrundem Querschnitt entsteht ein hoher Kontaktdruck zwischen Kontaktformstück 12 und Stift 13. Bei Verwendung solcher Bauteile sind vorzugsweise Mittel vorgesehen, um den Stift 13 in der Kontaktposition (nach dem Verdrehen) zu sichern.

**Ansprüche**

1. Steckernegativ für elektrischen Steckverbinder, insbesondere für miniaturisierte Bauteile, zur Aufnahme eines als Stift ausgebildeten mindestens einpoligen Steckerpositivs, an dessen Aussenfläche wenigstens eine Leiterfläche vorgesehen ist, wobei das Stekkernegativ für den jeweiligen Kontakt mit der Leiterfläche bzw. den Leiterflächen eines Steckerpositivs ausgebildet ist, dadurch gekennzeichnet, dass das Steckernegativ ein Isolatorgehäuse aufweist, welches wenigstens ein leitendes Anschluss- bzw. Kontaktelement trägt und das Ganze mit einer ggf. durchgehenden Ausnehmung zur Aufnahme des Stiftes eines Steckerpositivs versehen ist, wobei jedes Anschluss- bzw. Kontaktelement mit wenigstens einem in die Ausnehmung ragenden Kontaktformstück aus elektrisch leitenden Elastomeren verbunden ist.

2. Steckernegativ nach Anspruch 1, zur Aufnahme eines Steckerpositivs mit mehreren elektrisch voneinander getrennten Leiterflächen, dadurch gekennzeichnet, dass mehrere, z.B. zwei elektrisch voneinander getrennte leitende Anschluss- bzw. Kontaktelemente vorgesehen sind.

3. Steckernegativ nach Anspruch 1 oder 2, dadurch gekennzeichnet, dass jedes Kontaktformstück als scheiben- oder ringförmiges Element mit einem Durchgang bzw. einer Ausnehmung zur Aufnahme des Stiftes eines Steckerpositivs ausgebildet ist.

4. Steckernegativ nach Anspruch 2 oder 3, dadurch gekennzeichnet, dass die Kontaktformstük- ke hintereinander angeordnet sind und dass deren Durchgänge bzw. Ausnehmungen zueinander ausgerichtet sind.

5. Steckernegativ nach einem der Ansprüche 1-4, dadurch gekennzeichnet, dass der freie Querschnitt der Ausnehmungen in den Kontaktformstück kleiner ist als der kontaktwirksame Querschnitt des aufzunehmenden Stiftes des Steckerpositivs.

6. Steckernegativ nach einem der Ansprüche 1-5, dadurch gekennzeichnet, dass der Querschnitt der Ausnehmungen in den Kontaktformstücken unrund, z.B. vieleckig ausgebildet ist.

7. Steckernegativ nach einem der Ansprüche 1-6, dadurch gekennzeichnet, dass die Kontaktformstücke aus elektrisch leitendem Silicon-Elastomer, z.B. aus einem mit leitendem amorphem Kohlenstoff dotierten, hochtemperaturvulkanisierten Silicon-Elastomer bestehen.

8. Steckerpositiv für elektrischen Steckverbinder mit einem wenigstens eine Leiterfläche aufweisenden Kontaktstift, zur Verwendung mit einem Steckernegativ nach einem der Ansprüche 1-8, dadurch gekennzeichnet, dass der Kontaktstift einen unrunden, z.B. einen vielekkigen Querschnitt auf- weist.

# Fig.1

# Fig. 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| P,X | EP-A-0339877 (MEDTRONIC,INC.)<br>* Zusammenfassung; Figur 2 *<br>--- | 1-5, 7 | H01R13/24<br>H01R17/18 |
| X | US-A-4712557 (HARRIS)<br>* Spalte 5, Zeilen 24 - 48; Figuren 4, 5 *<br>--- | 1-5, 7 | |
| A | DE-A-2059875 (MULTI-CONTACT AG.)<br>* Anspruch 1; Figur 1 *<br>----- | 8 | |

**RECHERCHIERTE SACHGEBIETE (Int. Cl.5 )**

H01R

Der vorliegende Recherchenbericht wurde für alle Patentansprüche erstellt

| Recherchenort | Abschlußdatum der Recherche | Prüfer |
|---|---|---|
| DEN HAAG | 22 OKTOBER 1990 | HORAK A. L. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
........................................................................
& : Mitglied der gleichen Patentfamilie, übereinstimmendes
    Dokument

EPO FORM 1503 03.82 (P0403)